(19) Europäisches Patentamt
European Patent Office
Office européen des brevets

(11) Publication number: 0 119 190 B1

(12) # EUROPEAN PATENT SPECIFICATION

(45) Date of publication of patent specification: 27.04.88

(51) Int. Cl.$^4$: C 07 C 37/16

(21) Application number: 82902971.9

(22) Date of filing: 15.09.82

(86) International application number:
PCT/US82/01246

(87) International publication number:
WO 84/01146 29.03.84 Gazette 84/09

(54) **Catalyst composition for the Ortho-Alkylation of a phenol.**

(43) Date of publication of application:
26.09.84 Bulletin 84/39

(45) Publication of the grant of the patent:
27.04.88 Bulletin 88/17

(84) Designated Contracting States:
DE FR GB NL

(56) References cited:
CA-A- 907 065
US-A-3 446 856
US-A-3 843 606
US-A-3 873 638
US-A-3 968 172
US-A-3 971 832
US-A-3 972 836
US-A-3 974 229
US-A-4 041 085
US-A-4 100 207
US-A-4 201 880

**No relevant documents have been disclosed.**

**The file contains technical information submitted after the application was filed and not included in this specification**

(73) Proprietor: GENERAL ELECTRIC COMPANY
1 River Road
Schenectady New York 12305 (US)

(72) Inventor: BENNETT, James, Gordy
16 East Bayberry
Glenmont, NY 12077 (US)
Inventor: TUNGATE, Freddie, Lee
Rt. 1, Box 18
Georgetown, IN 47122 (US)

(74) Representative: Schüler, Horst, Dr. European Patent Attorney et al
Kaiserstrasse 41
D-6000 Frankfurt/Main 1 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The alkylation of the ortho position of phenols having at least one ortho-hydrogen is known in the art. Such alkylation is usually carried out in the presence of a catalyst.

Hamilton, U.S. 3,446,856 discloses a method for methylating the ortho positions of phenol by the vapor phase reaction of a phenol with methanol in the presence of magnesium oxide at a temperature in the range of 475° to 600°C.

Van Sorge, U.S. 3,873,628, discloses a method for the ortho-alkylation of phenol in the presence of a catalyst consisting of a mixture of magnesium oxide and manganese sulfate.

Other Van Sorge patents describe methods for the ortho-alkylation of phenols using a magnesium oxide catalyst together with a "promoter" consisting of manganese oxide (U.S. 3,974,229), or a magnesium oxide catalyst bonded with an inert organic cellulosic polymeric binder (U.S. 3,972,828).

It is desirable to carry out the orthoalkylation of phenols in the presence of a catalyst which does not depend on the use of a promoter compound or polymeric binders for the catalyst. The procedure described in the aforementioned Hamilton patent, using magnesium oxide alone serves the purpose to some extent. However, it is known that in Hamilton's procedure the service life of the magnesium oxide catalyst is relatively short because of the high temperature required, 475° to 600°C, and there is only moderate selectivity with respect to methanol.

Starting from this prior art it is the object of this invention to provide a catalyst composition for the orthoalkylation of a phenol consisting of the residue derived from calcining magnesium carbonate, basic magnesium carbonate or magnesium hydroxide, which allows the reaction to proceed at lower temperatures than permitted by many prior art processes, without a reduction in selectivity to the desired ortho-alkylated end product.

The catalyst of the present invention is characterized in that it is the residue derived from the calcination of magnesium carbonate, basic magnesium carbonate, magnesium hydroxide or mixtures of the foregoing at 400°C to 460°C in the presence of a vaporized ortho-alkylation feed mixture comprising a phenolic compound having at least one ortho-hydrogen and an alkyl alcohol.

By the term "magnesium carbonate" there is meant those compounds having the formula $MgCO_3$, as well as those compounds known as "basic" or "light" magnesium carbonates, e.g., having the formula $4MgCO_3 \cdot Mg(OH)_2 \cdot 5H_2O$.

The catalyst composition prepared in situ is used for the production of ortho-alkylated phenols by the vapor phase reaction of an alkyl alcohol and a phenol having at least one unsubstituted ortho-position.

Examples of the alcohols include saturated aliphatic alcohols such as methyl-, ethyl-, propyl-, isopropyl-, butyl-, isobutyl-, tert-butyl-, amyl-, isoamyl-, hexyl-, heptyl-, octyl-, nonyl-, decyl-, lauryl-, cetyl-, cyclohexyl-, and the like, alcohols, especially alcohols having up to 6 carbon atoms, and most preferably, methanol.

The invention is described with reference specifically to phenols and ortho-cresol. It can be applied in general to any phenol having an ortho-hydrogen, however. For instance, the process can be used with ortho-phenyl phenol, ortho-ethyl phenol, and phenols in which there are alkyl and aryl groups in the meta- and para-positions. These phenols can be represented by the formula:

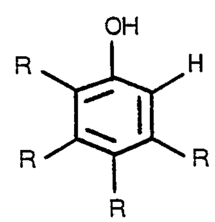

where each R is a monovalent substituent selected from the group consisting of hydrogen, alkyl, e.g. $C_1$—$C_{12}$ alkyl, phenyl and alkyl-substituted phenyl, e.g., $C_1$—$C_{12}$ alkyl-substituted phenyl.

In carrying out an alkylation any one or a mixture of phenols having an ortho hydrogen is vaporized and passed through a reactor heated to a temperature of at least 400°C and containing the magnesium carbonate and/or magnesium hydroxide catalyst of the invention.

For best yields of the desired ortho-alkylated products, it is preferred to use at least one mole of the alkyl alcohol, and preferably from 1 to 3 moles, for each ortho position on the phenol to be alkylated. For example, if phenol, which has two ortho hydrogens per molecule, is to be methylated to produce a maximum yield of 2,6-xylenol, it is desirable to use two to six moles of methanol for each mole of phenol, with higher yields being obtained with higher ratios of methanol to phenol.

The vapors issuing from the reactor are condensed and the products are separated by conventional methods such as crystallization, distillation, etc. The reaction proceeds at atmospheric pressure, but pressures above or below can be used as desired.

The selectivity favoring ortho alkylation over meta or para alkylation of the phenol is good, and usually better than what is achievable with promoted or bound magnesium catalysts. Yields of 90% or greater are

typical. These results are unexpected in view of prior art teachings which require promoters or polymeric binders for catalysts.

The invention is further illustrated by the examples which follow.

Preparation of Catalyst:

A magnesium carbonate ($MgCO_3$) catalyst for use in the invention is prepared. For purposes of comparison, three ortho-alkylation catalysts in accordance with the prior art are also prepared. The procedures are described below.

$MgCO_3$ catalyst (in accordance with invention)

About 225 grams of $MgCO_3$, Merck Chemical Co. commercial grade, is thoroughly wet with distilled water by agitation in a beaker. The $MgCO_3$ is then filtered on a fritted filter and the wet filter cake is dried in a pan in a vacuum oven. The dried cake is ground through a #25 mesh screen and 200 grams of this powder are put in a jar. Then 1.0 gram of graphite is added and the two powders are rolled on a jar mill for one hour to produce a 99.5:0.5 blend of $MgCO_3$: graphite.

This blend is pre-compressed by tabletting on a Betapress and the tablets are ground using a mortar and pestle and sieved through the #25 mesh screen. The resulting denser powder is tabletted into the final tablets for use in the ortho-alkylating procedure.

$MgCO_3$ + MnOx (Comparison catalyst A)

This catalyst is made by first slurrying commercial grade $MgCO_3$, Merck in distilled water. Then $Mn(NO_3)_2$, 50% commercial grade, is dropped into the slurry after dilution in distilled water. The ratio of $MgCO_3$ to $Mn(NO_3)_2$, by weight, is 11.33/1, or 0.02 moles of $MN(NO_3)_2$ per moles of $MgCO_3$. This slurry is then stirred for four hours under nitrogen gas in a closed environment, at a temperature of 82°C.

After four hours the slurry is filtered and dried. The resulting powder, on analysis, is found to contain 1.0% Mn. Three hundred grams of the powder are blended with 1.5 grams of graphite by rolling on a jar mill for one hour. The 99.5/0.5 blend which results is tabletted on a Betapress. Precompression is necessary, so these first tablets are ground with a mortar and pestle and sieved through a #25 mesh screen. The resulting denser powder is tabletted into the final tablets for use in the ortho-alkylating procedure.

$MgCO_3$ + Polyphenylene ether (Comparison catalyst B)

Approximately 225 grams of $MgCO_3$, Merck commercial grade is first thoroughly wet with distilled water and then filtered on a fritted filter. The wet cake is dried in a vacuum oven and the dried cake is ground through a #25 mesh screen. 200 grams of this powder are put in a jar and 22.22 grams of poly(2,6-dimethyl-1,4-phenylene ether) (PPO, General Electric Co.) are added to this and the two powders are blended by rolling on the jar mill for 15 minutes. Thus, a 90:10 blend of $MgCO_3$:PPO is created. Then 1.11 grams of graphite (Asbury A-99) are added to the jar and the three powders are rolled on the jar mill for one hour. This results in a 99.5:0.5 ratio of $MgCO_3$ + PPO:graphite. This blend is then precompressed, by tabletting, on the Betapress due to the lightness of the powder. The precompressed tablets are then ground with a mortar and pestle and sieved through the #25 screen. This denser powder is then tabletted into the final tablets for use in the ortho-alkylating procedure.

$MgCO_3$ + MnOx + Polyphenylene ether (Comparison catalyst C)

This catalyst is made by slurrying commercial grade $MgCO_3$, Merck in distilled water. Then $Mn(NO_3)_2$, 50% commercial grade is dropped into the slurry after dilution in distilled water. The ratio of $MgCO_3$ to $Mn(NO_3)_2$, by weight, is 11.3/1, or 0.02 moles of $Mn(NO_3)_2$ per mole of $MgCO_3$. This slurry is stirred for four hours under a $N_2$ blanket, in closed atmosphere, at a temperature of 82°C.

After four hours, the slurry is filtered and dried. This powder is determined to contain 1.0% Mn. 300 grams of this catalyst powder is then blended with 33.33 grams of PPO on the jar mill for 15 minutes to create a 90:10 blend of catalyst:PPO. Then 1.67 grams of graphite, Asbury A-99, are added to this and the powders are rolled on the jar mill for one hour to create a 99.5:0.5 blend of catalyst + PPO:graphite.

This blend is then precompressed on a Betapress. The tablets thus formed are ground with a mortar and pestle and sieved through the #25 screen. The denser powder thus obtained is then tabletted into the final tablets on the Betapress.

In the example, the reactor consists of two 19 mm (¾ inch) (I.D.) tubes. The feed, a solution of alcohol and phenol compound, is fed from a reservoir through a metering pump into the first of the two 19 mm (¾ inch) (I.D.) tubes which functions as a vertical vaporizer. The tube is 380 mm (15 inches) in length and is partially immersed to a depth of 203 mm (8 inches) in a fused salt bath. The vapors from the vaporizer are fed to the second 19 mm (¾ inch) (I.D.) tube, which functions as a vertical reactor, through a 50 mm (2 inch) length of stainless steel tubing located 127 mm (5 inches) above the bottom of the vaporizer and connected to the reactor tube 355 mm (14 inches) from its bottom. The reactor tube is 610 mm (24 inches) long and is immersed in the fused salt bath to a depth of 432 mm (17 inches).

The inlet tube of the reactor coming from the vaporizer also passes through the fused salt bath and

serves as a preheater for the vapor issuing from the vaporizer to bring the vapor up to the temperature of the reactor tube.

The second (reactor) 19 mm (¾ inch) (I.D.) tube is filled to a depth of 50 mm (2 inches) with glass beads which serve as support for the catalyst bed, and to a depth of 305 mm (12 inches) with 110 ml of catalyst. The vapors from the vaporizer are fed to the top of the catalyst bed and product vapors leave the bottom of the reactor tube through a 9.5 mm (⅜ inch) (I.D.) stainless steel outlet tube. The product vapors are led to a water-cooled condenser and receiver where they are liquified and recovered.

## Example

A $MgCO_3$ catalyst prepared as described above is evaluated in the reactor in a process according to the invention. After the reactor is charged with the catalyst and capped it is placed in a 370°C salt bath and a stream of gaseous nitrogen at a rate of 56 $dm^3$ (2 standard cubic feet) per hour is blown over the catalyst bed. After 15 minutes, the feed is started. The feed consists of 4/1 methanol/phenolics, with the phenolic being 60/40 phenol/ortho-cresol, and 20% water. The feed rate is 215 ml/hr, equivalent to a liquid having space velocity (LHSV) of 1.95 $hr^{-1}$. The LHSV defines the volume of liquid per volume of catalyst per hour. The rate is maintained for the duration of the run, 502 hours. The pressure is atmospheric.

Using the $MgCO_3$ catalyst, a temperature program is followed to maintain the desired conversion. After the feed is established at 370°C, the temperature is raised to 445°C which is reached, depending on the salt bath in use, in 1.5 to 2.5 hours. Listed below are the approximate time and temperature changes over the 502 hour run.

| Time, hours | Temp., °C |
|---|---|
| 0 | 370 |
| 0.5—19 | 450 |
| 19—502 | 455 |

The results near the mid-point and at the end of the run are reported below.

Phenolic Distribution

| Time, hours | Off Gas SCFH | Off Gas $dm^3$ | Phenol, wt.% | o-cresol, wt.% |
|---|---|---|---|---|
| 196 | 0.5 | 14.1 | 0.8 | 13.2 |
| 502 | 0.3 | 8.4 | 1.6 | 18.1 |
| TWA | 0.4 | 11.2 | 1.7 | 18.5 |

| Time, hours | 2,6-xylenol produced, wt.% | 2,4,6-trimethylphenol produced weight % |
|---|---|---|
| 196 | 80.4 | 5.3 |
| 502 | 75.5 | 4.5 |
| TWA | 73.7 | 5.5 |

For purposes of comparison, the same procedure is repeated, except that the $MgCO_3$ catalyst is substituted with comparison catalysts A ($MgCO_3$ + MnOx), B ($MgCO_3$ + PPO) and C ($MgCO_3$ + MnOx + PPO), respectively. The phenolic distribution data for each are given below.

Catalyst A ($MgCO_3$ + MnOx)

| Time, hours | Off Gas SCFH | Off Gas $dm^3$ | Phenol, wt.% | o-cresol, wt.% |
|---|---|---|---|---|
| 196 | 0.6 | 16.9 | 1.0 | 14.1 |
| 502 | 0.5 | 14.1 | 2.6 | 19.7 |
| TWA | 0.5 | 14.1 | 1.7 | 16.9 |

| Time, hours | 2,6-xylenol produced, wt.% | 2,4,6-trimethylolphenol produced weight % |
|---|---|---|
| 196 | 77.6 | 6.9 |
| 502 | 71.9 | 5.5 |
| TWA | 73.2 | 7.5 |

Catalyst B ($MgCO_3$ + PPO)

| Time, hours | Off Gas SCFH | Off Gas $dm^3$ | Phenol, wt.% | o-cresol, wt.% |
|---|---|---|---|---|
| 196 | 0.5 | 14.1 | 1.2 | 15.3 |
| 502 | 0.4 | 11.2 | 1.9 | 17.6 |
| TWA | 0.4 | 11.2 | 1.9 | 18.1 |

| Time, hours | 2,6-xylenol produced, wt.% | 2,4,6-trimethylphenol produced weight % |
|---|---|---|
| 196 | 77.7 | 5.5 |
| 502 | 75.5 | 4.7 |
| TWA | 73.3 | 6.0 |

Catalyst C ($MgCO_3$ + MNOx + (PPO)

| Time, hours | Off Gas SCFH | Off Gas $dm^3$ | Phenol, wt.% | o-cresol, wt.% |
|---|---|---|---|---|
| 196 | 0.6 | 16.9 | 2.8 | 20.2 |
| 502 | 0.4 | 11.2 | 6.3 | 25.4 |
| TWA | 0.5 | 14.1 | 4.5 | 23.8 |

| Time, hours | 2,6-xylenol produced, wt.% | 2,4,6-trimethylphenol weight % |
|---|---|---|
| 196 | 70.4 | 6.2 |
| 502 | 63.2 | 4.8 |
| TWA | 64.5 | 6.5 |

The overall results are summarized as follows:

| Run # | 1 | 2 | 3 | 4 |
|---|---|---|---|---|
| catalyst | $MgCO_3$ | $MgCO_3$+MnOx | $MgCO_3$+PPO | $MgCO_3$+MnOx+PPO |
| wt.% 2,6 | 73.7 | 73.2 | 73.3 | 64.5 |
| wt.% 2,4,6 | 5.5 | 7.5 | 6.0 | 6.5 |
| Off gas (SCFH)) $dm^3$ | (0.4) 11.2 | (0.5) 14.1 | (0.4) 11.2 | (0.5) 14.1 |
| Selectivity ratio | 13.4 | 9.8 | 12.2 | 9.7 |

It can be seen that run #1, using $MgCO_3$ catalyst above in accordance with the invention, produce superior results. The above data indicates that MnOx (run #2) is primarily responsible for increased off gas and decreased selectivity, both of which are undesirable. PPO (run #3) contributes slightly to decreased selectivity, probably due to a porosity effect. The use of both MnOx and PPO (run #4) is also inferior — run #1 results in 14% higher conversion, 16% lower off gas and 35% higher selectivity by comparison.

The above-mentioned patents and/or publications are incorporated herein by reference. Obviously, other modifications and variations of the invention are possible within the wording of the claims. For instance, instead of magnesium carbonate ($MgCO_3$), "basic" magnesium carbonate, magnesium hydroxide or a mixture of magnesium carbonate and magnesium hydroxide can be used as the catalyst.

**Claims**

1. A catalyst composition for the ortho-alkylation of a phenol, consisting of the residue derived from calcining magnesium carbonate, basic magnesium carbonate or magnesium hydroxide, characterized in that the catalyst is the residue derived from the calcination of magnesium carbonate, basic magnesium carbonate, magnesium hydroxide or mixtures of the foregoing at 400°C to 460°C in the presence of a vaporized ortho-alkylation feed mixture comprising a phenolic compound having at least one ortho-hydrogen and an alkyl alcohol.

2. A catalyst composition according to claim 1, in which the ortho-alkylation feed mixture comprises ortho-cresol, phenol and methanol.

**Patentansprüche**

1. Katalysator-Zusammensetzung für die ortho-Alkylierung eines Phenols bestehend aus dem durch Calcinierung erhaltenen Rückstand von Magnesiumcarbonat, basischem Magnesiumcarbonat oder Magnesiumhydroxid, dadurch gekennzeichnet, daß der Katalysator der Rückstand ist, der durch Calcinierung von Magnesiumcarbonat, basischem Magnesiumcarbonat, Magnesiumhydroxid oder Mischungen dieser vorgenannten Stoffe bei 400°C bis 460°C in Anwesenheit einer verdampften ortho-Alkylierungsausgangsmischung, die eine Phenolverbindung mit wenigstens einem ortho-Wasserstoff und einen Alkylalkohol enthält, erhalten wird.

2. Katalysator-Zusammensetzung nach Anspruch 1, in welcher die ortho-Alkylierungsausgangsmischung o-Kresol, Phenol und Methanol enthält.

**Revendications**

1. Une composition de catalyseur pour l'ortho-alcoylation d'un phénol, constituée du résidu dérivé de la calcination du carbonate de magnésium, du carbonate basique de magnésium ou de l'hydroxyde de

magnésium, le catalyseur étant caractérisé en ce qu'il est le résidu dérivé de la calcination du carbonate de magnésium, du carbonate basique de magnésium, de l'hydroxide de magnésium ou de mélanges de ceux-ci entre 400°C et 460°C en présence d'un mélange d'alimentation d'ortho-alcoylation vaporisé comprenant un composé phénolique ayant au moins un hydrogène en position ortho et un alcool alcoylique.

2. Une composition de catalyseur selon la revendication 1, dans laquelle le mélange d'alimentation d'ortho-alcoylation comprend de l'ortho-crésol, du phénol et du méthanol.